# EUROPEAN PATENT APPLICATION

(11) **EP 4 095 251 A1**
(43) Date of publication of application: **30.11.2022**
(21) Application number: 21176517.7
(22) Date of filing: 28.05.2021
(51) Int. Cl.: C12P 17/04

(54) **PROCESS FOR THE PRODUCTION OF 5-HYDROXYMETHYLFURFURAL**

(71) Applicant: Cascat GmbH, 94315 Straubing (DE)
(72) Inventor: PICK, André, 36179 Bebra-Breitenbach (DE)
(74) Representative: Aera A/S

(57) **Abstract**

A continuous process for the production of 5-hydroxymethylfurfural (HMF) comprising the steps of (a) converting at least one di-, oligo- or polysaccharide to fructose using a catalyst, whereby the conversion comprises at least one equilibrium shifting step; whereby a fructose stream is obtained; (b) optionally mixing the fructose stream obtained in step (a) with an organic solvent in a ratio of from 1:1 to 1:9; (c) feeding the fructose stream into a continuous fixed-bed reactor system; (d) converting the fructose present in the fructose stream into 5-hydroxymethylfurfural (HMF) at a temperature from 70°C to 160°C, whereby a HMF containing stream is obtained; (e) cooling down of the HMF containing stream obtained in step (d) to a temperature between 10 °C - 100°C is described.

## Description

The present invention relates to a continuous process for the production of 5-hydroxymethylfurfural (HMF), a composition comprising HMF, water, fructose and phosphoric acid and a composition comprising HMF and water obtainable by the process of the invention.

5-Hydroxymethylfurfural (HMF) represents one key intermediate substance readily accessible from renewable resources and is a suitable starting source for the formation of various furan monomers which are used for the preparation of non-petroleum-derived polymeric materials (AVA Biochem: Commercialising renewable platform chemical 5-HMF. Green Processing and Synthesis, 3(3), (2014) 235-236).

Current processes for HMF production refer to hexoses as substrate and are separated in several independent process steps. Hexose, in form of aldose (glucose, mannose etc.) and ketose (fructose, tagatose etc.) are often mentioned but commonly refer to glucose as the most abundant monosaccharide and fructose as the product of isomerization (Kuster, B. F. M., and HJC Der Van Steen. "Preparation of 5-hydroxymethylfurfural part I. dehydration of fructose in a continuous stirred tank reactor." Starch-Stärke 29.3 (1977): 99-103). Therefore, conversion of fructose (ketose) is preferred in the state of the art because it is assumed that glucose conversion to HMF is depending on an isomerization into fructose. Also glucose conversion often leads to furfural formation which is an unwanted side-product complicating down-streaming.

Therefore, when producing HMF fructose is produced first using an oligo- or polysaccharide as starting material which is broken-down into the monosaccharide glucose and the subsequent isomerization into fructose. Purification of fructose is done using chromatographic steps. An important factor in such a process represents the purity of fructose for the conversion into HMF to reduce possible side products. Fructose purity of ≥ 99% is preferred for conversion to reduce down-stream costs for HMF purification. While not being bound by theory, it is generally believed that fructose is converted to HMF via an acyclic pathway, although evidence also exists for the conversion to HMF via cyclic fructofuranosyl intermediate pathways. The conversion of HMF is generally understood as an intramolecular condensation reaction in which three moles of water are released per mole of monosaccharide building block. Like other condensations, this one is acid-catalyzed. It is known that various by-products are formed when using very strong acids or at high reaction temperatures. Apart from levulinic acid and formic acid, brown-black compounds occur in particular in quantities that are not negligible in some cases, which are known as humins. Regardless of the mechanism of HMF formation, the intermediate species formed during the reaction may in turn undergo further reactions such as condensation, rehydration, reversion and other rearrangements, resulting in a plethora of unwanted side products.

HMF and 2,5-disubstituted furanic derivatives have great potential in the field of intermediate chemicals from regrowing resources. Due to its various functionalities, it has been proposed that HMF could be utilized to produce a wide range of products such as polymers, solvents, surfactants, pharmaceuticals, and plant protection agents, and has been reported to have antibacterial and anticorrosive properties. HMF is also a key component, as either a starting material or intermediate, in the synthesis of a wide variety of compounds, such as furfuryl dialcohols, dialdehydes, esters, ethers, halides and carboxylic acids.

In addition, HMF has great potential as a biofuel, which are fuels derived from biomass and are considered promising alternatives to fossil fuels. HMF is also currently under investigation as a treatment for sickle cell anemia. In short, HMF is an important chemical compound and a method of synthesis on a large scale to produce HMF absent significant amounts of impurities, side products and remaining starting material has been sought for nearly a century.

Agricultural raw materials such as starch, cellulose, sucrose or inulin are inexpensive starting materials for the manufacture of hexoses, such as glucose and fructose. As shown above, these hexoses can in turn, be converted to HMF. The dehydration of sugars to produce HMF is well known. HMF was initially prepared in 1895 from levulose by Dull (Dull, G. Chem. Ztg. 1895, 19, 216) and from sucrose by Kiermayer (Kiermayer, J. Chem. Ztg. 1895, 19, 1003). However, these initial syntheses were not practical methods for producing HMF due to low conversion of the starting material to product (Newth, F. H. "The formation of furan compounds from hexoses." Advances in carbohydrate chemistry. Vol. 6. Academic Press, 1951. 83-106).

The purification of HMF has also proved to be a troublesome operation. On long exposure to temperatures at which the desired product can be distilled, HMF and impurities associated with the synthetic mixture tend to form tarry degradation products. Because of this heat instability, a falling film vacuum still must be used. Even in such an apparatus, resinous solids form on the heating surface causing a stalling in the rotor and frequent shut down time making the operation inefficient. Prior work has been performed with distillation and the addition of a nonvolatile solvent like PEG-600 to prevent the buildup of solid humin polymers (Cope, U.S. Pat. No. 2,917,520). Unfortunately, the use of polyglycols leads to the formation of HMF-PEG ethers (Kuster, B. F. M., and J. Laurens. "Preparation of 5-hydroxymethylfurfural part II. dehydration of fructose in a tube reactor using polyethyleneglycol as solvent." Starch-Stärke 29.5 (1977): 172-176.).

Although preparation of HMF has been known for many years, a method which provides HMF with good selectivity and in high yields has yet to be found. HMF is not stable, especially in aqueous systems, under the reaction conditions necessary for synthesis (acid pH, temperature increase) and, on the one hand, reacts, when polymerized, with itself and / or starting materials and intermediates for forming the so-called humines, which are soluble or insoluble, depending on the length of the chain and lead to a brown to black color of the reaction solution. Complications arise from the rehydration of HMF, which yields by-products, such as, levulinic and formic acids (Kuster, B. F. M. "5-Hydroxymethylfurfural (HMF). A review focusing on its manufacture" Starch-Stärke 42.8 (1990): 314-321.). Further complications may arise as a result of solvent selection. Water is easy to dispose of and dissolves fructose, but unfortunately, low selectivity and increased formation of polymers and humin increases under aqueous conditions.

Commonly used catalysts for the preparation of HMF include cheap inorganic acids such as H₂SO₄, H₃PO₄, and HCI (Kuster, B. F. M. "5-Hydroxymethylfurfural (HMF). A review focusing on its manufacture" Starch-Stärke 42.8 (1990): 314-321.). These acid catalysts are used in solution and are difficult to regenerate.

It is the object of the present invention to provide a method for the preparation of HMF starting from di-, oligo- and/or polysaccharides by combining fructose generation and conversion to HMF in one process.

This object is solved by the continuous process for the production of 5-hydroxymethylfurfural (HMF) according to the invention, the composition comprising HMF, water, fructose and phosphoric acid and a composition comprising HMF and water obtainable by the process according to the invention.

In a first aspect the invention relates to a continuous process for the production of 5-hydroxymethylfurfural (HMF) comprising, preferably consisting of, the steps of:
(a) converting at least one di-, oligo- or polysaccharide to fructose using a catalyst, preferably a biocatalyst, more preferably at least one enzyme,
   whereby the conversion comprises at least one equilibrium shifting step, preferably the equilibrium shifting step is catalyzed by at least one enzyme, preferably a phosphatase; whereby a fructose stream is obtained;
(b) optionally mixing the fructose stream obtained in step (a) with an organic solvent in a ratio of from 1:1 to 1:9 by volume;
(c) feeding the fructose stream into a continuous fixed-bed reactor system;
(d) converting the fructose present in the fructose stream into 5-hydroxymethylfurfural (HMF) at a temperature from 70°C to 160°C, whereby a HMF containing stream is obtained;
(e) cooling down of the HMF containing stream obtained in step (d) to a temperature between 10 °C to 100 °C and optionally adjusting the pH to 4.5 to 10.5.

The process comprises generation of fructose by converting di-, oligo- or polysaccharides and subsequent conversion of the fructose into HMF using said fructose without a purification step. The fructose process stream is optionally blended with an organic solvent and passed through a fixed-bed reactor; preferably the fixed-bed reactor contains a sulfonic acid type ion exchange resin. After passing the reactor a mixture containing a high amount of HMF and minor amounts of unreacted monosaccharides and side-products is obtained.

In the inventive process fructose generation and accumulation to higher concentrations with the help of an equilibrium shifting step and conversion of the process stream to HMF without intermediate purification steps are combined.

The advantage of the inventive process is that fructose is produced with high efficiency and less side-product accumulation such as e.g. glucose accumulation. This is possible through the at least one equilibrium shifting step. Shifting an equilibrium according to the present invention means that a (intermediary) product or another reactant is removed by a non-reversible step. In the present invention preferably an intermediary product (e.g. fructose-6-phosphate) obtained during the conversion of the at least one di-, oligo- or polysaccharide is removed from an equilibrium which forms during the conversion to fructose. By the non-reversible cleavage of the intermediary product (e.g. fructose-6-phosphate) the equilibrium shifts to produce more fructose, that is, the equilibrium is shifted in an equilibrium shifting step.

According to a preferred embodiment the equilibrium shifting step is catalyzed by at least one enzyme, preferably by a phosphatase.

It is preferred that the phosphatase comprises an amino acid sequence that is at least 80%, preferably at least 85%, more preferably at least 90%, even more preferably at least 97%, and most preferably at least 99 % identical with the sequence according to SEQ ID NO: 17.

It is further preferred that the phosphatase has an amino acid sequence according to SEQ ID NO: 13 or SEQ ID NO: 17.

Preferably the equilibrium shifting step in the conversion of the di-, oligo-, or polysaccharide to fructose only requires the addition of low amounts of phosphate salts and divalent metal ions, like magnesium, and required enzymes. In this case the subsequent steps are not negatively impacted.

The so obtained fructose in the obtained fructose stream can directly, without further purification, be converted to a HMF containing stream in a continuous fixed-bed reactor. Preferably the fructose stream is diluted with an organic solvent.

The final step in the conversion allows the stabilization of the obtained HMF.

Also several unexpected positive effects result from the inventive process. After conversion into fructose and dilution combined with an optional blending with an organic solvent passing this solution onto the continuous fixed-bed reactor an ion exchange resin can fully bind the cations of the fructose stream. In case phosphate salts are added the phosphate salts will be transferred into phosphoric acid and a system of solid/liquid acids will catalyze the dehydration reaction. After passing the continuous fixed-bed reactor system, which could for example be a column, the low amount of phosphoric acid will allow a fast stabilization of the HMF by a pH adjustment to neutral conditions (within a pH range of 6-8). Only low amounts of base will be required to achieve this pH adjustment and keeps the salt load low.

According to a preferred embodiment in step (a) at least two enzymes, preferably at least three enzymes and more preferably four to six enzymes are used as biocatalyst(s); and/or the temperature in step (a) is in the range from 10 to 100°C, preferably from 20 to 90°C, and more preferably form 20°C to 70°C.

The right choice of catalyst(s) makes it possible to obtain a high yield of fructose in the process by forming intermediate sugar-phosphates. As a result, a higher proportion based on the substrates (saccharides) can be achieved than in the currently used industrial production methods and a workup is made superfluous. This eliminates costly cleaning procedures.

In a preferred embodiment the catalyst(s), preferably the biocatalyst(s) and more preferably the enzyme(s) are immobilized (fixed-bed reactor) and the substrate stream passes the immobilized catalyst(s), preferably the biocatalyst(s) and more preferably the enzyme(s).

Preferably, the solution in step (a) is heated and passed through a fixed-bed reactor containing for example solid phase catalysts.

Further preferred the fructose stream obtained in step (a) contains phosphoric acid and/or a salt of phosphoric acid. Preferably the fructose stream obtained in step (a) contains phosphoric acid and/or a salt of phosphoric acid in an amount of from 0.01 to 200 mM, and more preferably 0.1 to 150 mM.

Preferably in step (a) at least one additional saccharide is added when oligo- or polysaccharides represent the starting substrate, whereby the at least one additional saccharide is selected from the group consisting of saccharides comprising 20 or less monosaccharide residues such as e.g. maltose, maltotriose, maltotetraose or maltodextrins, and/or combinations thereof. This allows for a faster conversion of the at least one oligo- or polysaccharide into fructose. When at least one disaccharide is the starting material in step (a) the addition of an additional saccharide is not required.

Preferably in step (a) saccharide phosphates are intermediary produced.

In a preferred embodiment in step (a) at least one transferase, preferably a glycosyltransferase, more preferably a glucanotransferase, even more preferably an alpha-glucanotransferase is added; and/or in step (a) at least one phosphorylase, preferably a glucanphosphorylase is added; and/or in step (a) at least one mutase, preferably a phosphoglucomutase is added; and/or in step (a) at least one isomerase, preferably a phosphoglucoisomerase is added; and/or in step (a) at least one hydrolase, preferably a glucanohydrolase, more preferably Pullulanase is added.

Preferably in step (a) the pH of the composition is in the range from 3 to 12, preferably from 4 to 10, and more preferably from 4.5 to 9.

The at least one di-, oligo- or polysaccharide converted in step (a) can for example be starch, cellulose, sucrose or inulin.

In a preferred embodiment sucrose is converted to fructose in step (a), yielding fructose concentrations of 160% or more based on the total amount of sucrose present in step (a). A disaccharide like e.g. sucrose is converted completely to fructose in a reaction media composed of water, phosphate buffer, small amounts of magnesium chloride and the biocatalysts. Preferably the temperature is the range from 20 to 90 °C, more preferably from 25 to 80 °C, and even more preferably from 30 to 80 °C.

Preferably step (a) is performed in a saccharide conversion unit, preferably a fixed-bed reactor. The saccharide conversion unit can for example be a column containing the required biocatalyst(s) in an immobilized form or alternatively also be a tank with the free biocatalyst(s), preferably the saccharide conversion unit is a column containing the required biocatalyst(s) in an immobilized form or a tank with the free biocatalyst(s).

Step (c) is performed subsequently after step (a) without purification. This means, the obtained fructose stream is not subjected to an additional purification step.

After step (a), preferably after leaving the fixed-bed reactor, the high concentrated fructose stream may be mixed with a solvent, preferably an organic solvent, in a ratio of from 1:1 to 1:9 by volume, in optional step (b). Preferably, the reaction solution in step (b) is monophasic and comprises water and an organic solvent in a ratio of 1:1 to 1:9 by volume.

The organic solvent is preferably an aprotic organic solvent or a mixture of at least two aprotic organic solvents. Preferably the aprotic organic solvent is selected from the group consisting of acetone, methylisobutylketone, tert.-butyl methyl ether (MTBE), 1,4 dioxane, dimethylformamide (DMF) or mixtures thereof. Preferably the organic solvent is a mixture of acetone/methylisobutylketone/MTBE/1,4-dioxane.

Preferably in step (b) the organic solvent is added in amount of 50 to 95 % by volume of the initial fructose solution.

Organic solvents including aprotic polar solvents are preferred because they are miscible with water, which helps with the solubility of fructose and with removing water. An example of a polar aprotic solvent is acetone as mentioned above, which is used to wash the wet resin and dehydrate the wet resin before the reaction. The resulting dehydrated resin is then dried under a vacuum prior to the reaction. In addition, DMF which is miscible with water and can be used as well as a solvent to dehydrate the wet resin. The dehydration of the wet resin may include raising the temperature of the reaction or any suitable method for dehydrating the wet resin or a combination thereof.

The fructose stream resulting from step (a) or the blended fructose stream resulting from step (b) is fed in step (c) into the fixed-bed reactor.

Fructose generation in a range of 100 mM to 2000 mM, preferably in a range of 200 mM to 1800 mM, more preferably in a range of 300 mM to 1700 mM and most preferably in a range of 400 mM to 1600 mM, allows the addition of acetone after passing the biocatalysts part. Acetone/methylisobutylketone/MTBE/1,4-dioxane is added to a 50 to 85 % by volume to the solution to reach a 1.8 - 3.6 wt.% fructose content. The formation of by-products depends on the initial concentration of the monosaccharide solution: the higher this concentration is, the higher are the formation rates for the by-products. Starting from a high concentration for the conversion and production of fructose allows in the subsequent steps to blend the reaction mix with an organic solvent to decrease the fructose concentration and also to reduce the water content for the HMF generation. Both are important factors to decrease side product formation and keep the specificity high for HMF.

According to a preferred embodiment step (b) is performed subsequently after step (a) without purification. This means, the obtained fructose stream is not subjected to an additional purification step.

According to another preferred embodiment step (d) is performed in a solid acid catalyst reactor.

Preferably in step (d) a solid catalyst of the type of an ion exchange resin, preferably an acidic ion exchange resin, and more preferably of the type of macroporous sulfonic acid ion exchange resins in a water/solvent mixture is used. The phosphoric acid acts as an acid catalyst next to the acidic ion exchange resin, preferably the solid macroporous sulfonic acid. Besides, the phosphoric acid and its respective salts also allow after the conversion process a fast pH adjusting for stabilization of produced HMF.

Further preferred in step (d) a solid catalyst of the type of macroporous sulfonic acid ion exchange resins is used. Other solid phase catalysts such as clays and zeolites can also be used.

A small-sized resin type in step (d) is preferred to reduce contact times which improve HMF yields and lower side product formation like humins and levulinic acid.

The use of solid phase catalysts in a chromatography column to synthesize HMF limits exposure time to heat and acid catalysts and enables synthesis at a lower temperature. Lower temperatures result in reduced energy costs and reduced time for heating and cooling the reaction. Non-limiting examples of solid phase catalysts that may be used in the process include acidic resins such as Amberlyst 35, Amberlyst 15, Amberlyst 36, Amberlyst 70, Amberlyst 131 (Rohm and Haas); Lewatit S2328, Lewatit K2431, Lewatit S2568, Lewatit K2629 (Lanxess); and Dianion SK104, PK228, RCP160, Relite RAD/F (Mitsubishi Chemical America, Inc.). Other solid phase catalysts such as clays and zeolites such as CBV 3024 and CBV 5534G (Zeolyst International), T-2665, T-4480 (United Catalysis, Inc), LZY 64 (Union Carbide), H-ZSM-5 (PQ Corporation) as described by Fleche, G.; Gaset, A.; Gorrichon, J. P.; Truchot, E.; Sicard, P. Fr. Patent Appl., 2464260, 1981; Rigal, Luc, et al. "Optimization of the conversion of d-fructose to 5-hydroxymethyl-2-furancarboxaldehyde in a water-solvent-ion exchanger Triphasic system-Part I. Investigation of the main effects of the major parameters and of their interactions on the reaction." Biomass 7.1 (1985): 27-45 can also be used. Acidic resins such as Amberlyst 35 are cationic, while catalysts such as zeolites, alumina, and clay are porous particles that trap small molecules. Soluble catalysts including inorganic acids, such as H₂SO₄, H₃PO₄, HCI, and organic acids such as p-toluene sulfonic acid may also be used. An advantage of solid phase catalysts is that they do not dissolve in solvent.

Because the synthesis of HMF is a dehydration reaction, a cation exchange resin having reduced water content is preferred. The presence of water in the reaction increases formation of by-products, such as, polymers and humins. If the water content of the wet resin is greater than about 20%, a solvent that is miscible with water may be selected as the solvent for the reaction in order to remove water from the wet resin.

Next to reduce the water content also the fructose concentration is reduced which helps to improve the conversion and minimizes side-product accumulation.

The temperature of the HMF containing stream obtained in step (d) is cooled in step (e) to a temperature from 10 to 100°C, preferably from 10 to 80°C; and more preferably from 10 to 70 °C.

Preferably the temperature of the HMF containing stream obtained in step (d) is cooled in step (e) to a temperature from 10 to 100°C, preferably from 10 to 80°C; and more preferably from 10 to 70 °C, and the pH is adjusted to 4.5 to 10.5.

Further preferred in step (e) the pH of the composition is adjusted in the range from 5 to 9, preferably from 6 to 8 using the phosphoric acid and/or a salt of phosphoric acid used in the process.

In another aspect the invention relates to 5-Hydroxymethylfurfural (HMF) obtainable by the process according to the invention.

All embodiments and features mentioned above in respect with the inventive process also apply to the 5-Hydroxymethylfurfural (HMF) obtainable by the inventive process.

In a further aspect the invention relates to a composition comprising 5-Hydroxymethylfurfural (HMF), water, fructose and phosphoric acid.

Regularly the composition comprises only low amounts of unreacted fructose.

In another aspect the invention relates to a composition comprising 5-Hydroxymethylfurfural (HMF) and water obtainable by the inventive process.

All embodiments and features mentioned above in respect with the inventive process also apply to the 5-Hydroxymethylfurfural (HMF) obtainable by the inventive process.

In the following the invention is further described by the figures:
- Fig. 1: Schematic representation of the inventive process starting with di-/oligo-/polysaccharide for fructose generation and the subsequent mixing with an organic solvent and feeding into the next conversion module for HMF generation; I: di-,oligo-, polysaccharide conversion unit (SCR), II: solvent reservoir, III: solid acid catalyst reactor (SACR), M: mixer unit for solvent addition;
- Fig. 2: Schematic representation of the inventive process; the conversion of starch (polysaccharide) into HMF;
- Fig. 3: intermediate step of the process shown in Fig. 2; glucan phosphorylase (3) using inorganic phosphate (G) to cleave an α-1,4 linkage between the terminal glucose residue and the rest of the polymer (B-A) to release glucose-1-phosphate (C);
- Fig. 4: intermediate step of the process shown in Fig. 2; glucose-1-phosphate (C) is converted by the action of a phosphoglucomutase (4) to glucose-6-phosphate (D);
- Fig. 5: intermediate step of the process shown in Fig. 2; glucose-6-phosphate (D) is converted to fructose-6-phosphate (E) using a phosphoglucoisomerase (5);
- Fig. 6: intermediate step of the process shown in Fig. 2; fructose-6-phosphate (E) is cleaved into fructose (F) and phosphate (G) by the action of a phosphatase (6);
- Fig. 7: intermediate step of the process shown in Fig. 2; conversion of fructose (F) into 5-hydroxymethlyfurfural (J) whereby water is released;
- Fig. 8: intermediate step of the process shown in Fig. 2; all α-1,6 linkages contained in starch (A - mixture of amylose (B) and amylopectin (H)) are cleaved by use of a Pullulanase (1);
- Fig. 9: intermediate step of the process shown in Fig. 2; α-Giucanotransferase (2) catalyzes the transfer of a segment of a 1,4- α -D-glucan (B) to a new position in an acceptor carbohydrate (B) generating a new 1,4-alpha linkage;
- Fig. 10: diagram showing the progress of the optimized process using an improved phosphatase, and maltose (0.1 mM) for faster conversion of native starch within 48 hours;
- Fig. 11: diagram showing the results of an experimental setting proofing the beneficial impact of short oligosaccharides for the breakdown of native starch using Pullulanase, α-Glucanotransferase and glucan phosphorylase and detection of the produced G1P;
- Fig. 12: diagram showing the results of an experimental for the conversion of sucrose into fructose using the equilibrium shifting process.

In the following the abbreviations and the enzymes used in the figures and/or tables are explained:
- I: Saccharide Conversion Unit (SCR)
- II: Solvent reservoir
- III: Solid Acid Catalyst Reactor (SACR)
- M: Mixer Unit for solvent addition
- 1: Pullulanase
- 2: α-Glucanotransferase
- 3: Glucan phosphorylase
- 4: Phosphoglucomutase
- 5: Phosphoglucoisomerase
- 6: Phosphatase
- 7: Macroporous sulfonic acid type ion exchange resin & phosphoric acid
- A: Starch
- B: Amylose
- B-A: Amylose minus 1 Glucose unit
- C: Glucose-1-Phosphate (G1P)
- D: Glucose-6-Phosphate (G6P)
- E: Fructose-6-Phosphate (F6P)
- F: Fructose
- G: Phosphate
- H: Amylopectin
- J: 5-Hydroxymethylfurfural

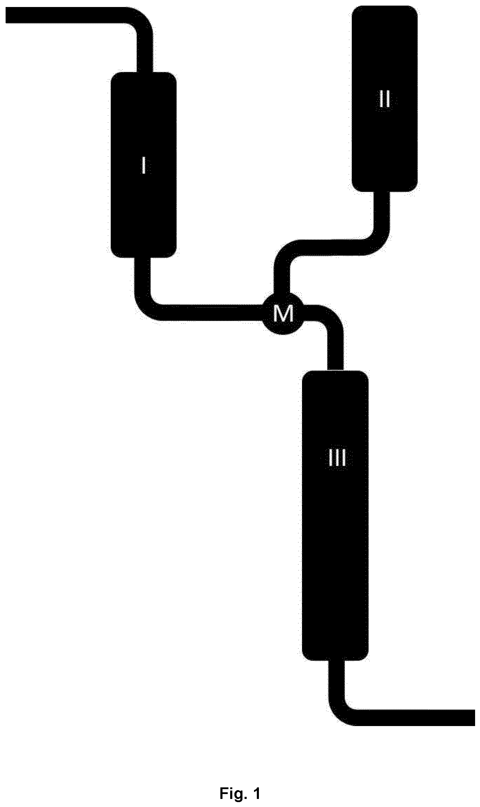

Fig. 1 shows the complete setup of the process. In the first reactor compartment (I) the di-, oligo-, polysaccharide solution is converted to fructose with an equilibrium shifting step which allows the accumulation of high fructose concentrations. This solution is directly fed into the second reactor compartment (III) or optionally blended with an aprotic organic solvent to reduce the water content. In the second reactor compartment (III) the accumulated fructose of the first conversion step is dehydrated using a mixture of liquid/solid acid catalysts consisting of a solid phase catalyst, like a macroporous sulfonic acid type ion exchange resin and phosphoric acid derived of the phosphoric acid salt added to the first reaction step for fructose accumulation. By passing the second reactor compartment (III) fructose is converted into HMF and the process stream is released. After the second reactor compartment (III) a HMF enriched process stream is generated. To stabilize the HMF the phosphoric acid in the process stream can be used as a buffer by adding small amounts of base, for example sodium or potassium hydroxide, to adjust the pH to a neutral level.

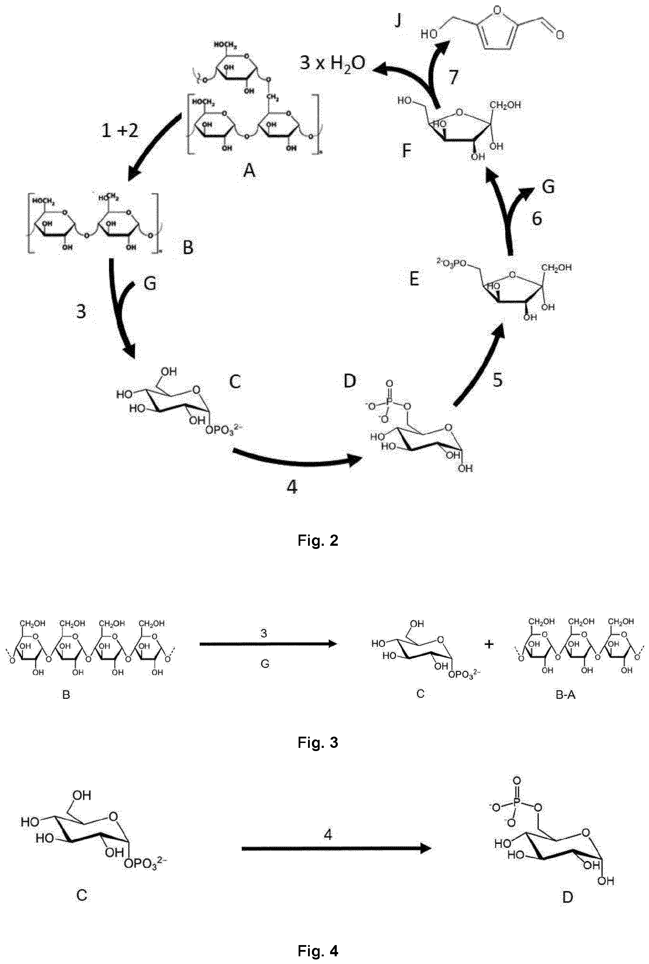

Fig. 2 shows the schematic representation of the inventive process starting with starch (A - mixture of amylose and amylopectin) which is converted to amylose (B) by use of pullulanase (1) and α-Glucanotransferase (2), the following step is catalyzed by a phosphorylase (glucan phosphorylase (3)) using inorganic phosphate (G) to cleave an α-1,4 linkage between the terminal glucose residue and the rest of the polymer to release glucose-1-phosphate (C), C is converted by the action of a phosphoglucomutase (4) to glucose-6-phosphate (D), glucose-6-phosphate is converted to fructose-6-phosphate (E) using a phosphoglucoisomerase (5) in the last step fructose-6-phosphate is cleaved into fructose (F) and phosphate (G) by the action of a phosphatase (6), finally the fructose is converted to HMF by dehydration in a separate reactor.

Fig. 3 shows an intermediate process step of the general process shown in Fig. 2. Glucan phosphorylase (3) and inorganic phosphate (G) are used to cleave an α-1,4 linkage between the terminal glucose residue and the rest of the polymer (B-A) to release glucose-1-phosphate (C).

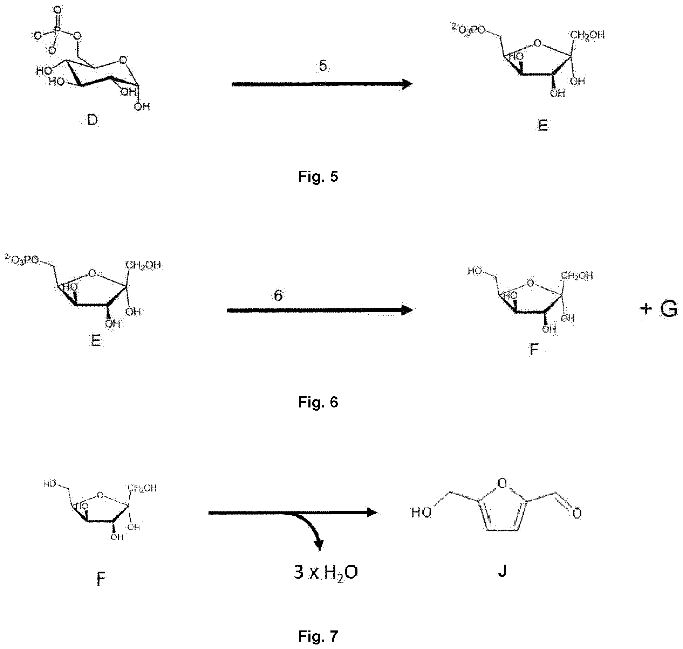

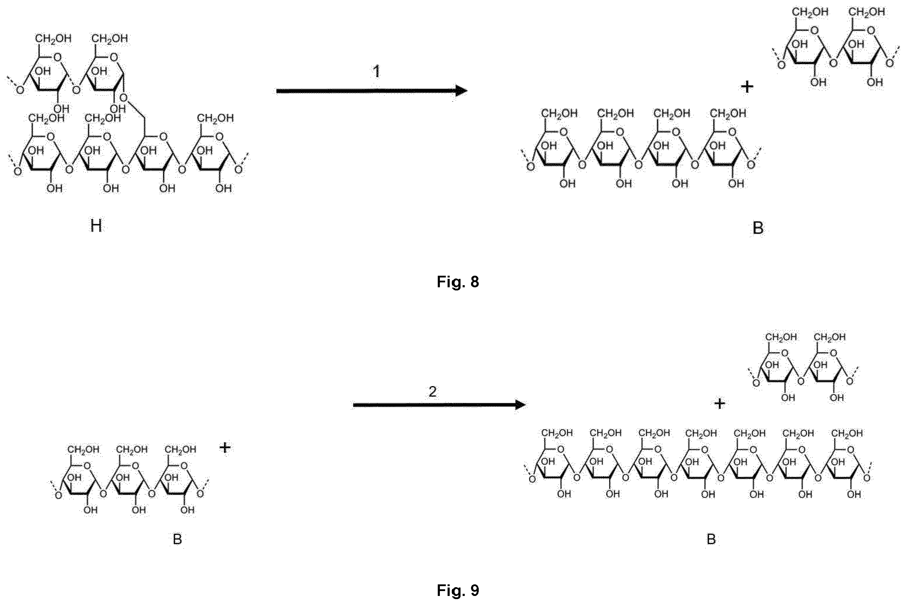

Fig. 4 to Fig. 9 show further intermediate steps of the process shown in Fig. 2.

### Experimental Section

### 1. Materials

All chemicals were of analytical grade or higher quality and purchased from Sigma-Aldrich, Carbosynth or VWR and used without further purification.

Glucidex 12 and Glucidex 19 are maltodextrin compounds which were obtained from Roquette Freres (France). The molecular mass of a glucose polymer can be calculated by using the formula (180*n - 18*(n-1)) with n the DP (degree of polymerization) of the glucose polymer. The DE (dextrose equivalent) can be calculated as 100*(180 / Molecular mass (glucose polymer)). The DP can be calculated using the equation DP = 111/DE (Balto, Amy S., et al. "On the use of differential solubility in aqueous ethanol solutions to narrow the DP range of food-grade starch hydrolysis products." Food chemistry 197 (2016): 872-880.). Glucidex 12 with a DE between 11 - 14 an average DP of 8 - 10 is assumed. Glucidex 19 with a DE between 18 - 20 an average DP of 5 - 6 is assumed. But as these preparations are derived by enzymatic treatment there are smaller and larger maltodextrins also included.

The following strains were used during this work; *E. coli* XL1 Blue, *E. coli* BL21 (DE3), every construct was successfully expressed using autoinduction media at 37 °C.

### 2. Methods

### Analytics

Analytics for the complete process as shown in Fig. 2 were performed using a HPLC Dionex Ultimate 3000 system equipped with autosampler (WPS 3000TRS), a column compartment (TCC3000RS) and a light scattering detector. The YMC Triart Diol Hilic column (100 * 2 mm, 1.9 µm, 12 nm) at 7 °C was used for separation by gradient elution (15-85 %) with 0.1% formic acid pH 4.5, and 0.1 % formic acid in acetonitrile as the mobile phase at 0.45 ml min⁻¹. Samples were diluted in water/acetonitrile with the ratio of 3:7. Data were analyzed using Dionex Chromeleon software.

Detection of overall ketose (fructose and fructose-6-phosphate) was done using an assay based on triphenyltetrazolium chloride (TTC). The detection method is based on a differential rate of reduction between aldoses and ketoses. Into a 2 ml test tube is added 0.05 ml sample, plus 0.01 ml 1% aqueous TTC, plus 0.04 ml 6 N NaOH. After exactly 5 min, 1.5 ml acetic acid:ethanol (1:9) is added and the test tube contents vortexed. With water used as a blank, absorbance is measured at 480 nm, using a spectrophotometer (Multiskan GO, Thermo Fisher). Glucose and glucose-6-phosphate reduces TTC to a red pigment--a triphenylformazan - about 100 times slower than an equivalent amount of fructose.

Glucose is detected using an assay based on glucose oxidase. 50 µl of the sample or diluted sample is mixed with 50 µl of master mix (0.75 mM ABTS, 2 U/ml glucose oxidase, 0.1 U/ml Peroxidase, 20 mM KPi pH 6.0) and incubated for 30 min at 30 °C and then measured at 418 nm using a spectrophotometer (Multiskan GO, Thermo Fisher).

### Protein expression

Optimized protein expression is exemplarily described for one enzyme and was performed for all proteins by the same procedure.

*E. coli* BL21 (DE3) containing the plasmid of interest (pET28 derivative with one gene encoding one of the described enzymes for the process) was grown in 250 ml autoinduction media (Studier, F. William. "Protein production by auto-induction in high-density shaking cultures." Protein expression and purification 41.1 (2005): 207-234.). The preculture was incubated in 4 ml of LB medium with 100 µg/ml kanamycin at 37 °C overnight on a rotary shaker (180 rpm). Expression culture was inoculated with a 1:100 dilution of overnight culture. Incubation was performed for 24 h at 37 °C. Cells were harvested by centrifugation and resuspended in 50 mM TRIS-HCI (pH 8.0). Crude extracts were prepared by use of a Basic-Z Cell Disrupter (IUL Constant Systems) or an ultrasonic device and subsequent addition of MgCl₂ to a final concentration of 2.5 mM in combination with DNasel (1 µg/ml) and a following incubation for 20 min at room temperature (25 °C) to degrade DNA. Afterwards all crude extracts were heat treated for 30 min at 70 °C. The insoluble fraction of the lysate was removed by centrifugation (20,000 rpm for 40 min at 4 °C). The supernatant was filtered through a 0.45 µm syringe filter and used as purified enzyme preparation. Aliquots of each purification were subjected to 12 % SDS-Page described by Laemmli Ulrich K. "Cleavage of structural proteins during the assembly of the head of bacteriophage T4." nature 227.5259 (1970): 680.).

The enzymes correspond to the following sequences SEQ ID NO: 1, SEQ ID NO: 3, SEQ ID NO: 5, SEQ ID NO: 7, SEQ ID NO: 9, SEQ ID NO: 11, SEQ ID NO: 13, SEQ ID NO: 15, and SEQ ID NO: 17.

The enzyme sucrose phosphorylase from *Bifidobacterium adolescentis* (SEQ ID NO: 15.) was expressed like described in the paragraph above and subsequent cells were harvested by centrifugation and resuspended in 50 mM sodium phosphate buffer (pH 8.0, 20 mM imidazol, 500 mM NaCl and 10 % glycerol). Crude extracts were prepared by use of a cell disrupter (IUL Instruments) and subsequent addition of MgCl₂ to a final concentration of 2.5 mM in combination with DNase (1 µg/ml) and a following incubation for 20 min at room temperature for DNA degradation. The insoluble fraction of the lysate was removed by centrifugation at 20,000 rpm for 40 min at 4 °C. The supernatant was filtered through a 0.45 µm syringe filter and applied to an affinity resin column, 5 ml HisTrapTM FF, equilibrated with the resuspension buffer using the ÄKTA UPC-900 FPLC-system. The enzyme was washed with 20 ml of resuspension buffer and eluted with 50 mM sodium phosphate buffer (pH 8.0, 500 mM imidazol, 500 mM NaCl and 10 % glycerol). Aliquots of each eluted fraction were subjected to 12 % SDS-Page. The fractions containing the eluted protein were pooled and the protein was desalted using a HiPrepTM 26/10 Desalting column which preliminary equilibrated with 50 mM TRIS-HCI pH 8.0. Aliquots of each purification were subjected to 12 % SDS-Page described by Laemmli Ulrich K. "Cleavage of structural proteins during the assembly of the head of bacteriophage T4." nature 227.5259 (1970): 680.).

### Activity testing of enzymes

Pullulanase of *Bacillus flavocaldarius* (SEQ ID NO: 1) was tested for activity using pullulan as substrate and 3,5-dinitrosalicylic acid (DNS) for detection.

Pullulanase was incubated with 1 % (w/v) pullulan in 50 mM potassium phosphate buffer pH 7.0 and 5 mM MgCl₂ at 65 °C. At different time points an aliquot of 24 µl was transferred into 96 µl of DNS reagent (10 g 3,5-dinitrosalicylic acid, 300 g sodium potassium tartrate, 16 g sodium hydroxide for 1 liter). Heated up to 95 °C for 5 minutes and afterwards were 100 µl transferred into a flat bottom microtitre plate and measured at 540 nm in a spectrophotometer (Multiskan GO, Thermo Fisher).

α-Glucanotransferase of *Meiothermus ruber* (SEQ ID NO:3) was tested for activity using maltotriose as substrate and the ability to build up larger oligosaccharides. Detection of activity was done using TLC (Isopropanol:Ethylacetate:Water (3:1:1)) and thymol spray reagent (0.5 g thymol 95 ml ethanol 5 ml sulfuric acid 97 %). α-Glucanotransferase of *Meiothermus ruber* was incubated with 50 mM maltotriose in 50 mM potassium phosphate buffer pH 7.0 and 5 mM MgCl₂ at 65 °C.

α-Glucan phosphorylase of *Thermotoga naphthophila* (SEQ ID NO: 5) was tested for activity using a combination of enzymes to detect the product α-D-glucose-1-phosphate. α-D-Glucose-1-phosphate was converted to α-D-glucose-6-phosphate which was subsequently oxidized by α-D-glucose-6-phosphate dehydrogenase to α-D-gluconate-6-phosphate. The produced NADH was used to convert WST-1 by using 1-Methoxy-5-methylphenazinium methyl sulfate (PMS) as electron mediator. α-Glucan phosphorylase was incubated with 1 % (w/v) soluble starch in 50 mM potassium phosphate buffer pH 7.0 and 5 mM MgCl₂ and 0.1 mM Pyridoxal-5-Phosphate at 65 °C. At different time points an aliquot of 10 µl was transferred into 190 µl of detection solution containing phosphoglucomutase 0.1 U, α-D-glucose-6-phosphate dehydrogenase 0.1 U, 0.1 mM WST-1, 0.005 mM PMS. The mixture was incubated for 30 min and the measured at 440nm in a spectrophotometer (Multiskan GO, Thermo Fisher).

Sucrose Phosphorylase of *Bifidobacterium adolescentis* (SEQ ID NO: 15) was tested for activity using sucrose as substrate and 3,5-dinitrosalicylic acid for detection. In an assay using 3,5-dinitrosalicylic acid for detection only the product fructose produces a signal. Sucrose Phosphorylase was incubated with 100 mM sucrose in 100 mM potassium phosphate buffer pH 7.0 and 5 mM MgCl₂ at 50 °C. At different time points an aliquot of 24 µl was transferred into 96 µl of DNS reagent (10 g 3,5-dinitrosalicylic acid, 300 g sodium potassium tartrate, 16 g sodium hydroxide for 1 liter). Heated up to 95 °C for 5 min and afterwards were 100 µl transferred into a flat bottom microtitre plate and measured at 540 nm in a spectrophotometer (Multiskan GO, Thermo Fisher).

Phosphoglucomutase of *Saccharolobus sulfataricus* P2 (SEQ ID NO: 7) or *Clostridium thermocellum* (SEQ ID NO: 9) was tested for activity using α-D-glucose-1-phosphate as substrate and 3,5-dinitrosalicylic acid for detection. In an assay using 3,5-dinitrosalicylic acid for detection only the product α-D-glucose-6-phosphate produces a signal. Phosphoglucomutase was incubated with 50 mM α-D-glucose-1-phosphate in 50 mM potassium phosphate buffer pH 7.0 and 5 mM MgCl₂ at 65 °C. At different time points an aliquot of 24 µl was transferred into 96 µl of DNS reagent (10 g 3,5-dinitrosalicylic acid, 300 g sodium potassium tartrate, 16 g sodium hydroxide for 1 liter). Heated up to 95 °C for 5 min and afterwards were 100 µl transferred into a flat bottom microtitre plate and measured at 540 nm in a spectrophotometer (Multiskan GO, Thermo Fisher).

Phosphoglucoisomerase of *Thermotoga maritima* (SEQ ID NO: 11) was tested for activity using fructose-6-phosphate as substrate and α-D-glucose-6-phosphate dehydrogenase for detection. The produced NADH was used to convert WST-1 by using 1-Methoxy-5-methylphenazinium methyl sulfate (PMS) as electron mediator. Phosphoglucoisomerase was incubated with 50 mM fructose-6-phosphate in 50 mM potassium phosphate buffer pH 7.0 and 5 mM MgCl₂ at 65 °C. At different time points an aliquot of 10 µl was transferred into 190 µl of detection solution containing α-D-glucose-6-phosphate dehydrogenase 0.1 U, 0.1 mM WST-1, 0.005 mM PMS. The mixture was incubated for 30 min and the measured at 440nm in a spectrophotometer (Multiskan GO, Thermo Fisher).

Optimized phosphatase of *Thermotoga naphthophila* (SEQ ID NO: 13) was tested for activity using fructose-6-phosphate as substrate and a phosphate assay. The phosphate assay consists of three solutions, solution A (12 % (w/v) L-ascorbic acid in 1N HCI solution), solution B (2 % (w/v) Na₂MoO₄ x 2H₂O in ddH₂O), solution F (2 % (w/v) citric acid and 2 % (w/v) acetic acid in ddH₂O). Solution D is a 2:1 mixture of solution A and solution B and prepared freshly before the measurement. 75 µl of solution D is pipetted into the needed wells of a flat-bottom microtiter plate. In the next step 25 µl of the sample or a standard with defined concentration is added and incubated for 5 min at room temperature. In the last step the reaction is stopped by addition of 75 µl of solution F and an additional incubation step of 15 min at room temperature. The samples are measured at 655 nm in a spectrophotometer (Multiskan GO, Thermo Fisher).

All tested enzymes fulfill the requirements to catalyze one of the steps within the process and are stable under process conditions (65 °C, 50 mM potassium phosphate buffer pH 7.0, 5 mM MgCl₂ 0.005 mM PLP).

### 3. Conversion of starch into fructose (Inventive Example 1; IE1)

Conversion experiments were performed at a 15 ml scale using 50 ml falcon tubes with 7 grams of native starch. Native starch was pretreated with a volume of 6 ml water and buffer and heated up to 95 °C for 30 min to allow an almost complete swelling. Afterwards, the swollen starch was cooled down to 65 °C and the enzymes were added. To 7 grams of native starch a volume of 10.5 ml of buffer, salts and enzymes was added. Additionally, maltose was added to a final concentration of 10 mM. The final mixture contained 50 mM KPi pH 7.0, 5 mM MgCl₂,0.1 mM PLP, 10 mM Maltose, 0.4 mg Pullulanase, 1.5 mg α-Glucanotransferase, 30 mg α-Glucan phosphorylase, 1.4 mg Phosphoglucomutase. 1.2 mg Phosphoglucoisomerase and 14 mg Phosphatase. Enzymes from *Bacillus flavocaldarius, Meiothermus ruber, Thermotoga naphthophila* (phosphorylase and phosphatase), *Clostridium thermocellum* and *Thermotoga maritima* were used. Starting with 0 hours' incubation on a regular basis samples were taken and filtered using a 10 kDa spin filter (VWR 82031-350). If necessary, samples were diluted in water/acetonitrile with the ratio of 3:7 for further analytics.

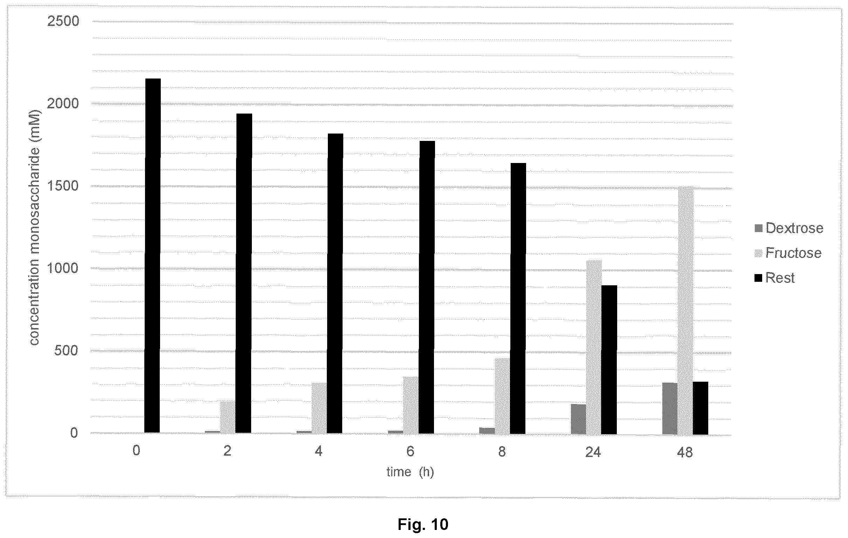

The results of the conversion of starch into fructose according to Inventive Example 1 (IE1) (35 % dry substance starch correspond to 2,153.5 mM concentration of monosaccharides) are shown in Table 1 as well as in Fig. 10.

**Table 1**

| | **Inventive Example 1** | | |
|---|---|---|---|
| **Time [h]** | **Fructose [mM]** | **Dextrose [mM]** | **Rest [mM]** |
| 0 | 0 | 0 | 2153.5 |
| 2 | 194 | 16 | 1943.5 |
| 4 | 312 | 18 | 1823.5 |
| 6 | 349 | 22 | 1782.5 |
| 8 | 464 | 40 | 1649.5 |
| 24 | 1059 | 186 | 908.5 |
| 48 | 1511 | 317 | 325.5 |

From Table 1 can be derived that after 48h fructose in a yield of about 70% is obtained.

### 4. Conversion of starch to fructose in the presence of maltose (IE2 - IE5), maltotriose (IE6 - IE9), maltotetraose (IE10 - IE12), Maltodextrin Glucidex D12 (IE13 - IE15) or Maltodextrin Glucidex D19 (IE16 - IE18)

Potassium phosphate buffer 50 mM pH 7.0, MgCl₂ 5 mM, PLP 0.005 mM, swollen native starch 1 % (w/v), Maltose (0.01 mM, 0.1 mM, 1.0 mM or 10 mM; IE2 - IE5) or Maltotriose (0.01 mM, 0.1 mM, 1.0 mM or 10 mM; IE6 - IE9) or Maltrotetraose (0.01 mM, 0.1 mM or 1.0 mM; IE10 - IE12) or Maltodextrin Glucidex 12 (0.0342 %(w/v), 0.00342 %(w/v) or 0.000342 %(w/v); IE13 - IE15) or Maltodextrin Glucidex 19 (0.0342 %(w/v), 0.00342 %(w/v) or 0.000342 %(w/v); IE16 - IE18), glucan phosphorylase (0.0025 mg/ml), alpha-glucanotransferase (0.075 mg/ml), pullulanase (0.02 mg/ml) in a total volume of 5 ml was incubated at 65 °C for one hour and aliquots were removed to check for glucose-1-phosphate. The glucose-1-phosphate assay is described under activity testing of enzymes above.

Comparative Examples 1 to 5 (CE1 - CE5) were performed by just adding 1mM of Maltose, Maltotriose, Maltotetraose, Glucidex 12 or Glucidex 19, respectively, without the addition of starch. Comparative Example 6 was performed without the addition of an additional saccharide such as maltose, maltotriose, maltotetraose, Glucidex 12 or Glucidex 19.

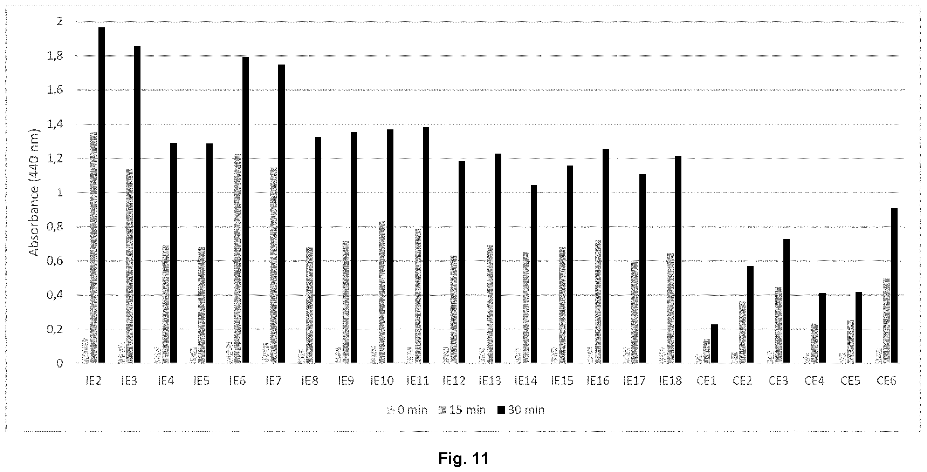

The results of the test on the influence of additional saccharides, such as short oligosaccharides like Maltose, Maltotriose, Maltotetraose, Glucidex 12 or Glucidex 19 on the conversion rate to glucose-1-phosphate is shown in Table 2. The respective diagram is shown in Fig. 11.

**Table 2**

| **Example (saccharide residue)** | **Absorbance at 440 nm** | | |
|---|---|---|---|
| | **at 0 [min]** | **at 15 [min]** | **at 30 [min]** |
| IE 2 (Maltose 10mM) | 0.147 | 1.351 | 1.966 |
| IE 3 (Maltose 1mM) | 0.125 | 1.138 | 1.857 |
| IE 4 (Maltose 0.1 mM) | 0.096 | 0.694 | 1.288 |
| IE 5 (Maltose 0.01mM) | 0.095 | 0.678 | 1.286 |
| IE 6 (Maltotriose 10mM) | 0.134 | 1.222 | 1.793 |
| IE 7 (Maltotriose 1 mM) | 0.120 | 1.148 | 1.748 |
| IE 8 (Maltotriose 0.1 mM) | 0.087 | 0.681 | 1.323 |
| IE 9 (Maltotriose 0.01 mM) | 0.094 | 0.713 | 1.352 |
| IE 10 (Maltotetraose 1 mM) | 0.101 | 0.832 | 1.369 |
| IE 11 (Maltotetraose 0.1 mM) | 0.097 | 0.784 | 1.383 |
| IE 12 (Maltotetraose 0.01mM) | 0.097 | 0.629 | 1.184 |
| IE 13 (Glucidex 12 0.0342 %(w/v)) | 0.092 | 0.689 | 1.226 |
| IE 14 (Glucidex 12 0.00342 %(w/v)) | 0.093 | 0.652 | 1.042 |
| IE 15 (Glucidex 12 0.000342 %(w/v)) | 0.094 | 0.679 | 1.159 |
| IE 16 (Glucidex 19 0.0342 %(w/v)) | 0.099 | 0.720 | 1.254 |
| IE 17 (Glucidex 19 0.00342 %(w/v)) | 0.093 | 0.595 | 1.106 |
| IE 18 (Glucidex 19 0.000342 %(w/v)) | 0.095 | 0.643 | 1.212 |
| CE 1 (only Maltose (1mM)) | 0.053 | 0.144 | 0.228 |
| CE 2 (only Maltotriose (1mM)) | 0.068 | 0.364 | 0.569 |
| CE 3 (only Maltotetraose (1mM)) | 0.079 | 0.446 | 0.728 |
| CE 4 (only Glucidex 12 0.0342 % (w/v)) | 0.063 | 0.237 | 0.413 |
| CE 5 (only Glucidex 19 0.0342 % (w/v)) | 0.065 | 0.255 | 0.418 |
| CE 6 (native starch) | 0.092 | 0.500 | 0.905 |

Conversion of large polysaccharides such as starch or comparable substrates like cellulose, and the like, within the process contains one major rate-limiting step, the generation of glucose-1-phosphate molecules catalyzed for example by a glucan phosphorylase. The phosphorylase needs accessible ends of the polysaccharide to catalyze cleavage of an α-1,4 linkage using inorganic phosphate between the terminal glucose residue and the rest of the polymer to release glucose-1-phosphate. The combined action of pullulanase, α-Glucanotransferase and short oligosaccharides such as maltose (IE2 - IE5), maltotriose (IE6-IE9), maltotetraose (IE10 - IE12), Maltodextrin Glucidex D12 (IE13 - IE15) or Maltodextrin Glucidex D19 (IE16 - IE18), cf. Table 2, prove that the conversion rate is increased by the addition of short oligosaccharides. This leads to a higher glucose-1-phosphate production and therefore allows a faster conversion of starch into fructose within the inventive process.

The conversion rate for all Comparative Examples 1 to 6 is lower in comparison to the Inventive Examples 2 to 18.

### 5. Phosphate Assays

For monitoring the activity of the phosphatase a phosphate assay was performed.

The reaction mixture contained 50 mM TRIS-HCI buffer pH 7.0, 2.5 mM MgCl₂, 10 mM of G1P, G6P or F6P and one phosphatase (0.003 mg/ml) in a total volume of 1 ml. The mixture was incubated at 65 °C and in regular time intervals an aliquot was removed and tested for released phosphate. The phosphate assay is described above in further detail.

### 6. Conversion of sucrose to fructose

Potassium phosphate buffer 50 mM pH 7.0, MgCl₂ 5 mM, sucrose 1000 mM, sucrose phosphorylase (0.25 mg/ml), Phosphoglucomutase. (0.04 mg/ml) Phosphoglucoisomerase (0.075 mg/ml) and Phosphatase (0.02 mg/ml) in a total volume of 10 ml were incubated at 50 °C for 25 hours and aliquots were removed to check for glucose and fructose concentration. Enzymes from *Bifidobacterium adolescentis (sucrose phosphorylase), Thermotoga naphthophila* (phosphatase), *Clostridium thermocellum* (phosphoglucomutase) and *Thermotoga maritima* (phosphoglucoisomerase) were used. Fructose and fructose-6-phosphate were determined as described in the methods.

In Table 3 the results of the conversion of sucrose to fructose is shown.

**Table 3**

| **Time** | **Fructose concentration [mM]** | | |
|---|---|---|---|
| | ***500mM Sucrose*** | ***600 mM Sucrose*** | ***700 mM Sucrose*** |
| 0 | 88.57 | 89.52 | 89.52 |
| 1 | 110.47 | 109.21 | 108.89 |
| 2 | 142.86 | 132.38 | 127.62 |
| 3 | 181.90 | 184.76 | 174.60 |
| 4 | 355.55 | 364.76 | 366.35 |
| 8 | 534.29 | 512.3.8 | 493.33 |
| 25 | 943.33 | 1124.39 | 1358.57 |

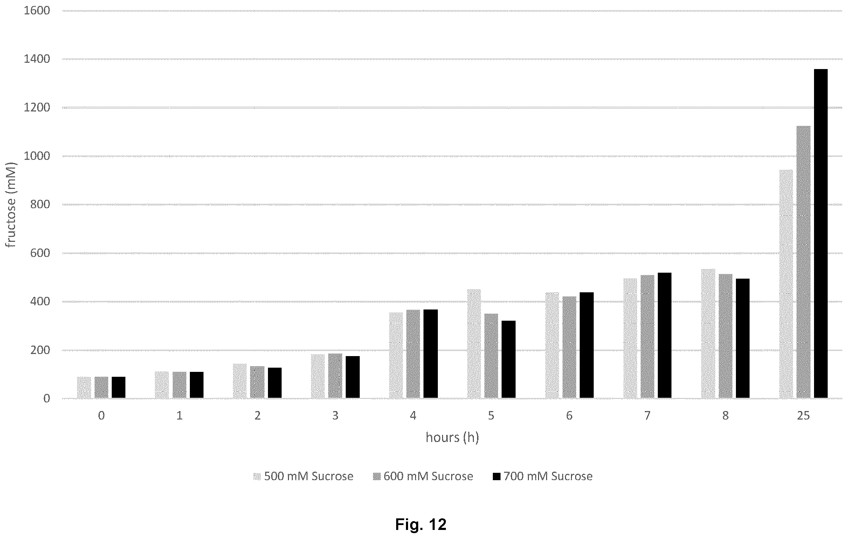

Conversion of different starting concentrations of sucrose (500 mM, 600 mM, 700 mM) into fructose (maximum concentration 1000 mM, 1200 mM, 1400 mM) using a combination of 4 enzymes: sucrose phosphorylase, phosphoglucomutase, phosphoglucoisomerase and phosphatase. The results are shown in Fig. 12.

### 7. Conversion of Fructose to HMF

In the first reactor compartment the di-, oligo-, polysaccharide solution is converted to fructose with an equilibrium shifting step which allows the accumulation of high fructose concentrations. In this example sucrose is converted into fructose using the 4 enzymes sucrose phosphorylase, phosphoglucomutase, phosphoglucoisomerase and phosphatase. Within 25 hours the complete sucrose is converted into fructose with minor amounts of glucose. After the conversion is completed the enzymes are removed by filtration against a 10kDa filter membrane and pumped into the next operation unit and blended with an aprotic organic solvent to reduce the water content. The mixing and dilution step with an aprotic organic solvent is not always required. In the second reactor compartment the accumulated fructose of the first conversion step is dehydrated using a mixture of liquid/solid acid catalysts consisting of a solid phase catalyst, like macroporous sulfonic acid type ion exchange resin and phosphoric acid derived of the phosphoric acid salt added to the first reaction step for fructose accumulation. The second reactor compartment is heated to a temperature between 70 and 160°C for up to several hours in accordance with Kuster, B. F. M., and HJC Der Van Steen. "Preparation of 5-hydroxymethylfurfural part I. dehydration of fructose in a continuous stirred tank reactor." Starch-Stärke 29.3 (1977): 99-103.; Nakamura, Yoshio, and Shunichi Morikawa. "The dehydration of D-fructose to 5-hydroxymethyl-2-furaldehyde." Bulletin of the chemical society of Japan 53.12 (1980): 3705-3706.). By passing the second reactor compartment the fructose is converted into HMF and the process stream is released. After the second reactor compartment a HMF enriched process stream is generated. To stabilize the HMF the phosphoric acid in the process stream can be used as a buffer by adding small amounts of base, for example sodium or potassium hydroxide, to adjust the pH to a neutral level.

The conversion can also be done as described by Mercadier *et al.* (Mercadier, Daniel, et al. "Synthesis of 5-hydroxymethyl-2-furancarboxaldehyde catalysed by cationic exchange resins. Part 2. Analysis and discussion of the effect of the main parameters on the HMF output." Journal of Chemical Technology and Biotechnology 31.1 (1981): 497-502

## Claims

1. A continuous process for the production of 5-hydroxymethylfurfural (HMF) comprising the steps of:
(a) converting at least one di-, oligo- or polysaccharide to fructose using a catalyst, preferably a biocatalyst, more preferably at least one enzyme,
whereby the conversion comprises at least one equilibrium shifting step, preferably the equilibrium shifting step is catalyzed by at least one enzyme, preferably a phosphatase;
whereby a fructose stream is obtained;
(b) optionally mixing the fructose stream obtained in step (a) with an organic solvent in a ratio of from 1:1 to 1:9 by volume;
(c) feeding the fructose stream into a continuous fixed-bed reactor system;
(d) converting the fructose present in the fructose stream into 5-hydroxymethylfurfural (HMF) at a temperature from 70°C to 160°C, whereby a HMF containing stream is obtained;
(e) cooling down of the HMF containing stream obtained in step (d) to a temperature from 10 °C to 100 °C and optionally adjusting the pH to 4.5 to 10.5.

2. Process according to claim 1,
whereby in step (a) at least two enzymes, preferably at least three enzymes and more preferably four to six enzymes are used as biocatalyst(s); and/or
whereby the temperature in step (a) is in the range of from 10°C to 100°C, preferably from 20°C to 90°C, and more preferably from 20°C to 70°C.

3. Process according to claim 1 or 2,
whereby step (b) is performed subsequently after step (a) without purification.

4. Process according to any of the preceding claims,
whereby in step (d) a solid catalyst of the type of macroporous sulfonic acid ion exchange resins is used; or
whereby in step (d) a solid catalyst of the type of macroporous sulfonic acid ion exchange resins in a water/solvent mixture is used.

5. Process according to any of the preceding claims,
whereby in step (b) the organic solvent is an aprotic organic solvent, preferably an aprotic organic solvent selected from the group consisting of acetone, methylisobutylketone, methyl tert-butyl ether (MTBE), 1,4-dioxane or mixtures thereof, more preferably a mixture of acetone, methylisobutylketone, methyl tert-butyl ether (MTBE) and 1,4-dioxane; and/or
whereby in step (b) the organic solvent is added in an amount of 50 to 95 % by volume of the initial fructose solution.

6. Process according to any of the preceding claims,
whereby the fructose stream obtained in step (a) contains phosphoric acid and/or a salt of phosphoric acid, preferably in an amount of from 0.01 to 200 mM.

7. Process according to any of the preceding claims,
whereby step (a) is performed in a saccharide conversion unit; and/or
whereby step (d) is performed in a solid acid catalyst reactor.

8. Process according to any of the preceding claims,
whereby in step (a) at least one additional saccharide is added, whereby the at least one additional saccharide is selected from the group consisting of saccharides comprising 20 or less monosaccharide residues and/or combinations thereof; and/or whereby in step (a) saccharide phosphates are intermediary produced.

9. Process according to any of the preceding claims,
whereby in step (a) at least one transferase, preferably a glycosyltransferase, more preferably a glucanotransferase, even more preferably an alpha-glucanotransferase is added; and/or
whereby in step (a) at least one phosphorylase, preferably a glucanophosphorylase is added; and/or
whereby in step (a) at least one mutase, preferably a phosphoglucomutase is added; and/or
whereby in step (a) at least one isomerase, preferably a phosphoglucoisomerase is added; and/or
whereby in step (a) at least one hydrolase, preferably a glucanohydrolase, more preferably Pullulanase is added.

10. Process according to any of the preceding claims,
whereby in step (a) the pH of the composition is in the range of from 3 to 12, and preferably from 4 to 10.

11. Process according to any of the preceding claims,
whereby the temperature in step (e) is in the range of from 10°C to 80°C; and preferably from 10°C to 70°C; and/or
whereby in step (e) the pH of the composition is adjusted in the range of from 5 to 9, preferably from 6 to 8 using the phosphoric acid and/or a salt of phosphoric acid used in the process.

12. Process according to any of the preceding claims,
whereby in step (a) sucrose is converted to fructose, yielding fructose concentrations of 160% or more based on the total amount of sucrose present in step (a), whereby preferably the temperature is in the range of from 20°C to 90 °C and more preferably from 30°C to 80 °C.

13. Process according to any of the preceding claims,
whereby the phosphatase comprises an amino acid sequence that is at least 80%, preferably at least 90% and more preferably at least 97% identical with the sequence according to SEQ ID NO: 17.

14. Composition comprising 5-Hydroxymethylfurfural (HMF), water, fructose, and phosphoric acid.

15. Composition comprising 5-Hydroxymethylfurfural (HMF) and water obtainable by the process according to claims 1 to 13.
